# DEMANDE DE BREVET EUROPEEN

(11) **EP 2 959 928 A1**
(43) Date de publication de la demande: **30.12.2015**
(21) Numéro de dépôt: 14174461.5
(22) Date de dépôt: 26.06.2014
(51) Int. Cl.: A61M 1/34, A61M 1/36

(54) **Dispositif d'épuration du sang par circulation extracorporelle**

(71) Demandeur: Infomed SA, 1227 Acacias (CH)
(72) Inventeur: Favre, Olivier, 1226 Thônex (CH)
(74) Mandataire: Micheli & Cie SA

(57) **Abrégé**

La présente invention a pour objet un dispositif d'épuration du sang comprenant une boucle de circulation extracorporelle du sang (1, 2, 3), des moyens de contrôle de débit (11) pour gérer la circulation du sang dans la boucle de circulation extracorporelle du sang (1, 2, 3), des moyens d'épuration du sang (5) agencés sur la boucle de circulation extracorporelle du sang (1, 2, 3), des moyens d'injection de citrate (4) agencés pour injecter une solution comprenant du citrate dans la boucle de circulation extracorporelle du sang (1, 2, 3) et une unité de commande (20) agencée pour commander lesdits moyens d'injection de citrate (4) et lesdits moyens de contrôle de débit (11). L'unité de commande (20) est en outre agencée pour requérir à des intervalles de temps déterminés d'un traitement d'épuration du sang mis en oeuvre par ledit dispositif la saisie d'au moins une valeur représentative de l'état du dispositif et/ou du patient. L'unité de commande (20) vérifie ensuite l'adéquation de ladite valeur représentative avec des règles préétablies enregistrées dans l'unité de commande (20) et commande lesdits moyens d'injection de citrate (4) et/ou les moyens le contrôle de débit (11) selon le résultat de ladite vérification et lesdites règles préétablies.

## Description

La présente invention se rapporte à un dispositif d'épuration extracorporelle du sang.

Il existe plusieurs méthodes d'épuration du sang pratiquées en fonction de critères décrits dans la littérature depuis plus de 40 ans. On peut notamment citer l'hémodialyse, l'hémofiltration, l'hémodiafiltration, l'échange plasmatique, la double filtration ou l'adsorption sur sang ou sur plasma extrait du sang. Ces moyens de traitement, généralement réalisés par un abord veino-veineux (le sang est pris et retourné dans une veine) sont largement décrits dans la littérature et peuvent, dans les cas cliniques les plus graves, être réalisés sous forme continue (24h/24). On les retrouve alors sous les dénominations : CVVHD (hémodialyse continue veino-veineuse), CVVH (hémofiltration continue veino-veineuse) et CVVHDF (hémodiafiltration continue veino-veineuse).

Toutes ces techniques, auxquelles peut s'appliquer le dispositif selon la présente invention, ont en commun de nécessiter une circulation extracorporelle de sang, soit une boucle de circulation de sang à l'extérieur des patients qui comprend des moyens de circulation du sang et un ou plusieurs éléments permettant l'échange avec le sang en vue de purifier ce dernier de substances en lien avec la pathologie. Du fait des propriétés coagulantes du sang, on ajoute souvent un anticoagulant dans le sang en amont de la boucle, avant l'élément d'échange. Les deux produits les plus habituels pour prévenir la coagulation du sang sont l'héparine et le citrate.

L'héparine, qui agit en activant l'antithrombine III, est généralement utilisée en anticoagulation systémique, c'est-à-dire que non seulement le sang est anticoagulé dans la boucle de circulation mais il le reste lorsqu'il retourne au patient ce qui entraine, entre autres, des risques hémorragiques.

Le citrate se lie lui au calcium, lequel est un élément incontournable du phénomène de coagulation. Il a été démontré qu'une concentration de calcium ionisé (Ca⁺⁺) dans le sang inférieure à 0.4 mmol/L de sang augmente le temps de coagulation, la courbe ayant une forme exponentielle et le sang ne coagulant plus à partir de 0.2 mmol/l (Kutsogiannis et Al., Regional citrate anticoagulation in continuous veinoveinous hemodiafiltration, American Journal of Kidney Diseases, Vol 35, No 5 (May), 2000:pp 802:811, Calatzis et Al., Citrate anticoagulation for extracorporeal circuits : Etfects on whole blood coagulation activation and clot formation, Nephron 2001 ;89:233-236). En pratique clinique, il a été démontré que réduire le calcium ionisé Ca⁺⁺ en dessous de 0.5 mmol/l permet de prolonger la vie du circuit de circulation extracorporelle (Nurmohamed et Al., Continuous veinoveinous hemofiltration with or without predilution regional citrate anticoagulation : A prospective study, Blood purification 2007;25:316-323).

Le calcium lié au citrate est en partie éliminé par toutes les techniques de filtration et dialyse citée précédemment, de même que le calcium qui reste ionisé. Il faut donc injecter du calcium au patient afin de compenser ces pertes et maintenir une calcémie « normale » du patient, de 1.1 à 1.3 mmol/L, afin de lui éviter une hypocalcémie. Ceci se fait le plus souvent sur la ligne de retour du sang et permet d'injecter au patient un sang ayant un calcium ionisé proche de celui extrait. On parle alors d'anticoagulation régionale puisqu'elle est limitée à la boucle de circulation extracorporelle, ce qui permet notamment par rapport à l'héparine de réduire les risques hémorragiques pour le patient (Park et Al., Regional anticoagulation with citrate is superior to systemic anticoagulation with heparin in critically ill patients undergoing continuous veinoveinous hamodialfiltration, original article, DOI: 10.3904/kjim.2011.26.1.68) et de prolonger le temps d'utilisation du circuit de circulation extracorporelle (Sheldon et Al., A novel regional citrate anticoagulation protocol for CRRT using only commercially available solutions, Journal of Critical care, Vol 18,No 2 (June), 2003: pp 121-129).

A noter qu'un modèle complet d'anticoagulation par injection de citrate doit aussi comprendre les liaisons du magnésium avec le citrate, les échanges entre le calcium ionisé Ca⁺⁺ et le calcium lié à l'albumine, la concentration de plaquettes, le taux d'hématocrite, le temps de coagulation, les variations de pH du patient ou encore la demi-vie du citrate dans le corps du patient. Des modèles ont été développés pour des cas spécifiques, par exemple (Kozik-Jaromin, *Citrate kinetics during regional citrate anticoagulation in extracorporeal organ replacement therapy,* Aus des Medizinischen Universitätklinik Abteilung Innere Medizin IV (Nephrologie ∼ 1 Allgemeinmedizin) der Albert-Ludwigs-Universität Freiburg i. Br., 2005) pour l'hémodialyse chronique d'une durée classique de quatre heures, afin d'évaluer à priori les doses de citrate et de calcium nécessaires à l'anticoagulation régionale du sang. Le brevet US 2011/0237996 décrit lui un appareil qui utilise des modèles mathématiques pour déterminer à priori les valeurs nécessaires et durant le traitement une correction basée sur des valeurs statistiques de parathormone et des phosphatases alcalines. Un tel appareil permet apparemment de traiter des patients atteints de défaillance rénale chronique, donc des patients stables qui viennent trois fois par semaine à l'hôpital et dont la situation clinique est bien connue du personnel soignant.

Cependant ces modèles aboutiraient souvent à des déviations importantes de concentration des solutés dans les cas de traitements continus, raison pour laquelle des brevets spécifiques à ces traitements existent. On peut citer le brevet US 2011/0168614 qui évoque les équations nécessaires à définir les entrées et sorties de citrate et de calcium principalement, mais aussi d'autres paramètres pouvant intervenir dans leur équilibre, pour tous les traitements de type continu (CVVHD, CVVH, CVVHDF) en se basant notamment sur les concentrations et les débits. Les auteurs prétendent pouvoir, sur la base de leur modèle de circulation des fluides extracorporels, piloter la pompe de calcium utilisée pour restaurer la calcémie du patient avant de lui retourner son sang. Le problème est ici que ce qui se passe dans le patient n'est pas pris en compte, notamment et à titre d'exemple le métabolisme du citrate injecté. Le brevet US 2011/0288464 remédie en partie à cela en proposant de piloter les pompes de citrate et/ou de calcium en se basant d'une part sur un modèle mathématique comparable à celui du brevet US 2011/0168614 et d'autre part en y ajoutant des mesures automatiques de paramètres tels que le débit du sang ou la concentration du calcium dans le circuit. Si un tel appareil va dans le bon sens, il ne comprend pas la situation clinique du patient puisque celle-ci n'est pas reflétée uniquement par des valeurs mesurables dans le sang mais également par d'autres paramètres comme par exemple sa situation cardiaque, inflammatoire ou respiratoire. Le choix des débits doit donc être de la responsabilité du personnel soignant et ne peut être confiée uniquement à l'appareil sur la base des valeurs mesurées par ce dernier et de règles de calcul. Ceci est vrai dans tous les cas de circulation extracorporelle mais l'est d'autant plus pour les patients soumis à des traitements continus du fait de leur situation clinique grave et complexe mais également du fait que cette situation évolue pendant le traitement.

En pratique, les nombreux protocoles de traitement utilisant une anticoagulation au citrate comportent d'une part des contrôles à intervalles de temps déterminés et d'autre part des corrections pas à pas en fonctions de valeurs mesurées et des valeurs visées données par le modèle mathématique utilisé. A titre d'exemple on peut voir les tables 2 et 3 du document Amanzadeh et Al., Anticoagulation and continuous renal replacement therapy, Seminar in dialysis, Vol 19, No 4 (July-August) 2006 pp.311-316 qui indiquent pour la première les recommandations de modification du débit de citrate en fonction du calcium ionisé Ca⁺⁺ mesuré après le filtre (postfiltre) et pour la seconde celles applicables au débit de calcium en fonction du calcium ionisé du patient. Dans les deux cas, pour chaque plage de valeur mesurée, une variation recommandée de débit est indiquée avec un délai avant le prochain contrôle et potentiellement le prochain ajustement. Par exemple, dans la table 2, si le Ca⁺⁺ est compris entre 0.41 et 0.45, on ajoute 20 ml/h au débit courant de citrate et on effectue la vérification suivante après une heure. Dans un autre exemple, le tableau 3 indique que si le calcium ionisé est au-delà de 1.35 mmol/L, le débit courant de calcium doit être réduit de 1.1 mmol/h, le prochain contrôle devant être effectué dans quatre heures.

Ces intervalles de temps peuvent être évalués en fonction de la situation présente et des modifications envisagées. En effet, plus la situation est proche de celle désirée, moins la modification éventuelle est importante et plus l'intervalle de temps avant le prochain contrôle est élevé car le risque pour le patient est faible. A l'inverse, si l'on se trouve dans une situation éloignée de celle souhaitée, l'intervalle de temps avant le prochain contrôle sera court car la correction et le risque pour le patient seront importants. En plus du risque pour le patient et de l'amplitude des modifications requises, l'intervalle de temps intègre le temps d'équilibre du système qui dépend notamment de la demi-vie du citrate, laquelle varie de trente minutes à deux heures en fonction notamment de la situation hépatique du patient.

Les exemples précédents montrent que les corrections de débits se font progressivement en fonction de l'écart entre le paramètre mesuré et sa valeur visée et que le résultat de ces corrections ne peut être vérifié qu'après un temps qui se mesure en heure soit un temps durant lequel le personnel soignant effectuera de nombreuses autres tâches. Il peut ainsi aisément laisser passer un délai ce qui entraîne des risques intrinsèques aux systèmes d'anticoagulation au citrate avec une compensation en calcium. Ces risques sont directement issus de quatre cas possibles que sont : trop ou trop peu de citrate et trop ou trop peu de calcium. Certains de ces risques peuvent entrainer une dégradation importante de l'état clinique du patient voire sa mort ; ils sont abondamment décrits dans la littérature (voir Davenport et Al., Citrate anticoagulation for continuous renal replacement therapy (CRRT) in patients with acute kidney injury admitted to the intensive care unit, NDT Plus (2009) 2:439-447) et souvent cités comme une limitation majeure à l'utilisation du citrate comme anticoagulant. Il paraît donc important de les limiter autant que possible mais les dispositifs d'épuration du sang par circulation extracorporelle qui utilisent du citrate comme anticoagulant, tels que décrits dans l'art antérieur, ne sont intrinsèquement pas sûrs du fait qu'ils n'obligent à aucune validation de l'adéquation de l'anticoagulation par le personnel soignant ni ne contrôlent, à des intervalles de temps adéquats, des valeurs de traitement saisies par ledit personnel en fonction de règles déterminées et de valeurs mesurées automatiquement, par exemple le calcium ionisé. De plus ils n'intègrent pas la tolérance maximale des patients au citrate, ni le temps de passage dans le circuit en fonction de son volume et du débit sang, ni les variations issues du métabolisme du citrate par le patient, trois paramètres pouvant avoir une influence majeure sur les risques encourus par le patient.

Le but de la présente invention est de remédier, au moins en partie, aux inconvénients susmentionnés et de réaliser un dispositif d'épuration du sang par circulation extracorporelle avec anticoagulation au citrate qui soit sûr et approprié pour une utilisation en traitement continu.

La présente invention a pour objet un dispositif selon la revendication 1.

La présente invention a donc pour objet un appareil qui permet de réaliser une anticoagulation au citrate qui, afin d'être sûre, requiert la connaissance à intervalles de temps validés d'au moins une valeur représentative de la situation de l'anticoagulation et utilise cette valeur pour autoriser, ou refuser, les variations de débits de traitements (sang, dialysat, substitution, perte de poids, citrate, calcium). L'intervalle de temps peut être variable, par exemple plus court en début de traitement ou après qu'un ou plusieurs paramètres aient été modifiés afin de mieux couvrir les périodes de stabilisation et réduire les risques pour le patient et plus long en régime stable c'est à dire lorsque le système ne subit pas de variations notamment lorsque la valeur représentative est proche de celle souhaitée, afin d'économiser du temps et de l'argent en évitant des mesures inutiles. Ceci participe également, au même titre qu'une anticoagulation bien gérée, à réduire les pertes de sang du patient ainsi que les risques de transfusion associés.

Les dessins annexés illustrent schématiquement et à titre d'exemple une forme d'exécution d'un dispositif d'épuration du sang par circulation extracorporelle selon l'invention.
La figure 1 illustre schématiquement un dispositif selon l'invention.
La figure 2 illustre schématiquement l'unité de commande d'un dispositif selon l'invention.
La figure 3 illustre schématiquement le fonctionnement du dispositif selon l'invention.

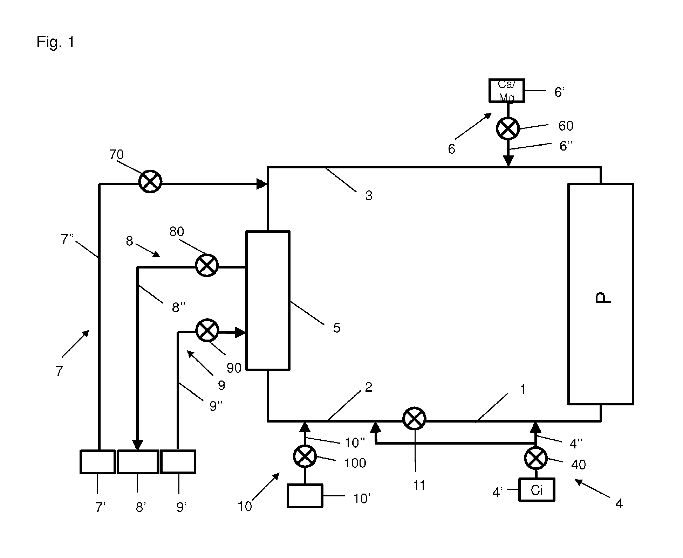

Un dispositif d'épuration selon l'invention comporte comme illustré sur la figure 1 une boucle de circulation extracorporelle du sang comprenant un conduit d'extraction 1, des moyens de circulation et de contrôle de débit du sang 11, comme une pompe, pour extraire le sang du corps du patient P, un conduit de retour 3 pour ramener le sang épuré dans le corps du patient P et des moyens de purifications du sang 5 qui sont placés entre un conduit intermédiaire 2 relié aux moyens de circulation du sang 11 en aval de ceux-ci et le conduit de retour 3 et qui sont constitués par exemple de filtres, dialyseurs ou cartouches d'adsorption.

Des moyens 4 permettant d'injecter un fluide contenant du citrate (Ci) sont placés sur la boucle de circulation du sang 1, 11, 2, 3. Ces moyens 4 comprennent une poche 4' contenant le fluide à injecter, un conduit 4" reliant la poche 4' au conduit d'extraction 1 ou au conduit intermédiaire 2 et une pompe 40 permettant d'injecter le contenu de la poche 4' dans le conduit d'extraction 1 ou le conduit intermédiaire 2 via le conduit 4".

De manière optionnelle, le dispositif peut être complété par des moyens 6 permettant l'injection de calcium (Ca) et/ou de magnésium (Mg) (soit par un élément unique comme illustré soit par des éléments séparés l'un pour le calcium et l'autre pour le magnésium) dans le conduit de retour 3, et par des moyens de traitement 7, 8, 9, 10 permettant de réaliser les techniques d'épuration connues. Les moyens d'injection du calcium/magnésium 6 peuvent comprendre notamment un conduit 6" reliant un réservoir 6' contenant une solution comprenant du calcium et/ou du magnésium au conduit d'extraction 3 et une pompe 60 pour l'injection du contenu du réservoir dans la boucle de circulation. Dans la figure 1, la référence 9 désigne notamment des moyens d'injection d'un dialysat comprenant un conduit dédié 9" relié aux moyens de purification du sang 5, un réservoir de dialysat 9' et une pompe 90 ; la référence 8 désigne des moyens d'extraction de la solution polluée comprenant notamment un conduit d'extraction 8" relié aux moyens de purification du sang 5, un réservoir pour le liquide pollué 8' et une pompe d'extraction 80 ; la référence 7 désigne des moyens d'injection de liquide de remplacement en postdilution comprenant un conduit de postdilution 7" relié au conduit d'extraction 3, une pompe de postdilution 70 et un réservoir de postdilution 7 ; et la référence 10 désigne des moyens d'injection d'un liquide en prédilution comprenant un conduit 10" relié au conduit intermédiaire 2 par exemple, un réservoir de prédilution 10' et une pompe de prédilution 100. Les moyens d'injection de dialysat 9 et d'extraction de la solution polluée 8 permettent notamment de réaliser une hémodialyse. Les moyens de prédilution 10, d'extraction de la solution polluée 8 et de postdilution 7 permettent de réaliser une hémofiltration et les moyens d'injection de dialysat 9, d'extraction de la solution polluée 8, de postdilution 7 et/ou de prédilution 10 une hémodiafiltration.

La boucle de sang 1, 2, 3 peut être complétée en pratique par les moyens habituels mais non représentés de protection du circuit, comme par exemple des capteurs de pression, ou du patient, comme par exemple un détecteur de fuite de sang ou un détecteur d'air associé à un clamp. Ces moyens sont largement connus de l'homme de l'art et décrits dans la littérature.

D'autres moyens de circulation et de contrôle de débit que les pompes 11, 40, 100, 90, 80, 70 et 60 mentionnées ci-dessus existent (par exemple des clamps) et sont décrits dans la littérature et peuvent évidemment être utilisés dans la présente invention à la place desdites pompes.

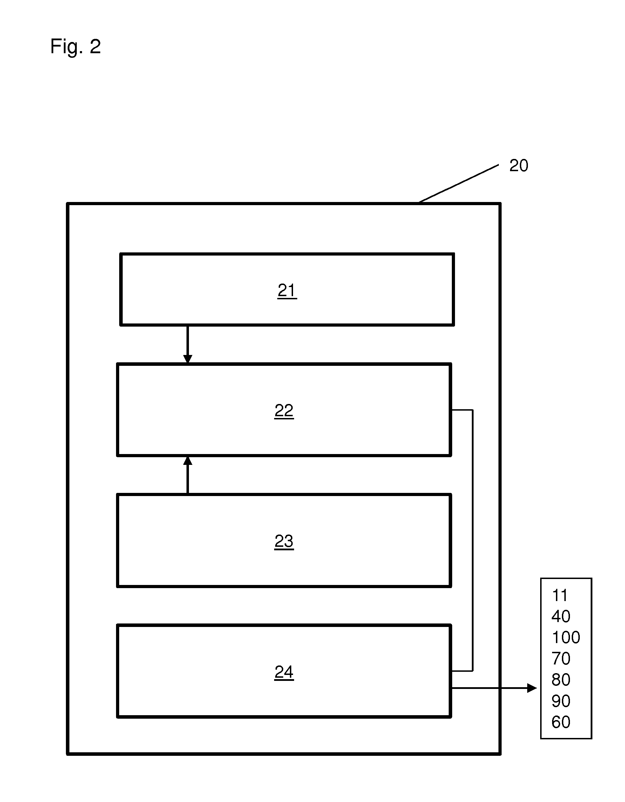

Le dispositif selon l'invention possède de plus une unité de commande 20 illustrée sur la figure 2 et comprenant notamment une interface utilisateur 21, une unité de calcul 22, une mémoire 23 et des moyens de commandes 24 reliés à chacun des moyens de circulation et de débit 11, 40, 60, 70, 80, 90, 100 présents sur la boucle de circulation du sang 1, 2, 3, 4 et permettant de commander lesdits moyens de circulation et de débit.

Selon l'invention, l'unité de commande 20 est programmée pour obliger l'utilisateur à insérer, via l'interface utilisateur 21, au moins un paramètre établi comme étant une valeur représentative et permettant le contrôle de l'adéquation des valeurs de traitement et d'anticoagulation, à intervalle de temps déterminé selon des règles préétablies et placées dans la mémoire 23.

Selon les modèles utilisés pour le traitement, la valeur représentative peut être par exemple le taux de calcium ionisé en un point de la boucle de circulation du sang 1, 2, 3, 4, le pH du patient ou son calcium total et toutes autres valeurs connues pour être représentatives dans le modèle considéré. Par la suite, l'expression « valeur représentative » désigne l'une quelconque de ces valeurs et/ou toute combinaison ou ensemble de ces valeurs. L'interface utilisateur 21 permet également la saisie par l'utilisateur des valeurs de traitement et d'anticoagulation et l'affichage des messages nécessaires au bon déroulement du traitement.

L'unité de calcul 22 vérifie les paramètres de traitement et d'anticoagulation requis, soit les débits des moyens de circulation 40, 60, 70, 80, 90, 100, 11, ou de certains d'entre eux, en fonction des règles préétablies et de la valeur représentative préalablement entrée par l'utilisateur et envoie à l'interface utilisateur les messages nécessaires pour la mise à jour du traitement. Si les valeurs requises sont acceptables pour l'unité de calcul 22, elle envoie alors les commandes correspondantes aux moyens de commandes 24 des moyens de circulation des fluides 40, 60, 70, 80, 90, 100, 11, ou de certains d'entre eux.

Les règles préétablies font appel à des équations connues mais peuvent aussi intégrer des conditions nouvelles, notamment des limites hautes et basses. Parmi les équations connues on peut notamment citer le fait qu'à chaque fluide correspond un débit « Q » représenté à titre d'exemple par les pompe(s) 40, 60, 70, 80, 90, 100, 11. Pour chaque substance contenue dans un fluide, la quantité injectée « M » par unité de temps « t » est donnée par le produit de la concentration « C » avec le débit correspondant soit : M = Q * C. Les débits et les concentrations étant connus de l'appareil, soit parce qu'ils sont saisis via l'interface utilisateur 21 soit parce qu'ils sont calculés automatiquement par l'unité de calcul 22, il est possible de calculer les masses des substances qui nous intéressent, notamment celles du citrate et du calcium, et d'en déduire des valeurs d'acceptation en appliquant des règles supplémentaires comme par exemple l'équilibre entre calcium injecté et perdu dans le circuit ou la masse de citrate maximale admissible. Les valeurs de débit et de concentration qui doivent être calculées le sont selon des règles connues de l'homme de l'art.

Pour illustrer ceci avec l'exemple d'un traitement d'hémofiltration l'opérateur fourni un débit de substitution en postdilution utilisé pour piloter la pompe 70 et un débit de perte de poids, le débit de rejet de la pompe 80 calculé est alors égal à la somme du débit de substitution et de la perte de poids afin de retirer du poids (en l'occurrence principalement sous forme d'eau) au patient en respectant la consigne de l'opérateur.

Un autre exemple serait le cas du calcul de la concentration de citrate dans le sang qui circule dans les moyens de purification 5 (Cci₅), en considérant que la pompe de prédilution 100 est arrêtée et n'intervient donc pas dans le calcul. Dans ce cas, la concentration serait calculée à partir de celle dans la poche de 4' (CCi₄) de fluide contenant du citrate et des débits de citrate QCi et de sang Qb avec : Cci₅ = CCi₄ * (QCi/(Qb + QCi)). On peut ainsi connaitre pour chaque point de la boucle de circulation 1, 2, 3 illustrés sur la figure 1, les débits et concentrations des substances qui nous intéressent soit parce qu'elles sont saisies via l'interface 21 par l'opérateur soit par calcul. Les calculs détaillés qui peuvent s'appliquer à la présente invention sous leur forme complète ou une autre simplifiée ont déjà fait l'objet de publications (voir Kozik-Jaromin, *Citrate kinetics during regional citrate anticoagulation in extracorporeal organ replacement therapy,* Aus des Medizinischen Universitätklinik Abteilung Innere Medizin IV (Nephrologie ∼ 1 Allgemeinmedizin) der Albert-Ludwigs-Universität Freiburg i. Br., 2005) et des brevets US 2011/0288464 et US 2011/0168614.

Pour rendre le traitement plus sûr, d'autres règles doivent également être appliquées, notamment celles qui définissent les valeurs limites de citrate. En effet, d'un côté le patient ne peut pas tolérer sans effets secondaires indésirables une injection de citrate au-delà d'une valeur établie, à 80 mg/kg/h dans la littérature, ou participant à un pH supérieur à 7.45 et de l'autre côté, une valeur trop faible qui augmenterait le risque de coagulation dans la boucle. Du fait d'un modèle incomplet qui veut que, le citrate se liant au calcium et au magnésium, plus il y a de sang plus il y a de calcium et de magnésium auquel se lier et donc plus il faut de citrate pour maintenir la valeur visée de calcium et le temps de coagulation, le débit de citrate est souvent défini comme un ratio du débit sang. Le problème à débit élevé de sang est que l'on injecte trop de citrate par rapport à ce que le patient peut tolérer et à ce qui est utile puisque, le temps dans le circuit diminuant lorsque le débit de sang augmente, l'anticoagulation peut être moindre à débit de sang élevé. A l'inverse, à bas débit de sang le temps dans le circuit est long et à débit de citrate proportionnel, donc anticoagulation constante, le risque de coagulation dans la boucle augmente alors même que le citrate injecté est loin en-dessous de la valeur limite acceptable pour le patient. Dans ce cas, on aurait donc intérêt à injecter proportionnellement plus de citrate dans le sang afin d'augmenter le temps de coagulation. Ainsi, le dispositif selon l'invention peut intégrer des règles et valeurs qui bornent la masse de citrate injectée, vers le haut en fonction de la tolérance du patient et vers le bas en fonction du temps de coagulation nécessaire pour que le sang parcours la boucle de circulation 1, 2, 3.

Aux règles d'évaluation décrites ci-dessus, un appareil selon l'invention ajoute des règles d'acceptation des paramètres saisis par l'opérateur via l'interface 21. Ces règles d'acceptation peuvent dépendre ou non du paramètre représentatif de l'anticoagulation. Par exemple un ratio de citrate élevé avec un débit de sang élevé aboutissant à une injection connue pour être dangereuse pour le patient peut être refusé, une limite supérieure de ratio de citrate pouvant alors être proposée. Un autre exemple est que si le paramètre représentatif est par exemple le calcium ionisé du patient, et que la valeur saisie montre une valeur connue pour être basse, par exemple inférieure à 1.1 mmol/L, le dispositif peut refuser que l'injection de calcium par la pompe 60 soit diminuée, ou ne l'accepter qu'après une double validation par l'opérateur. Les règles d'acceptation peuvent contenir, en fonction de la situation, une ou plusieurs valeur(s) optimale(s) et une marge de tolérance associée. On comprend rapidement qu'il existe la possibilité de créer de nombreuses règles d'acceptation et que celles-ci sont appelées à évoluer avec les connaissances de la médecine. Un dispositif selon l'invention peut ainsi utiliser toute règle visant à réduire les risques décrits précédemment pour le patient.

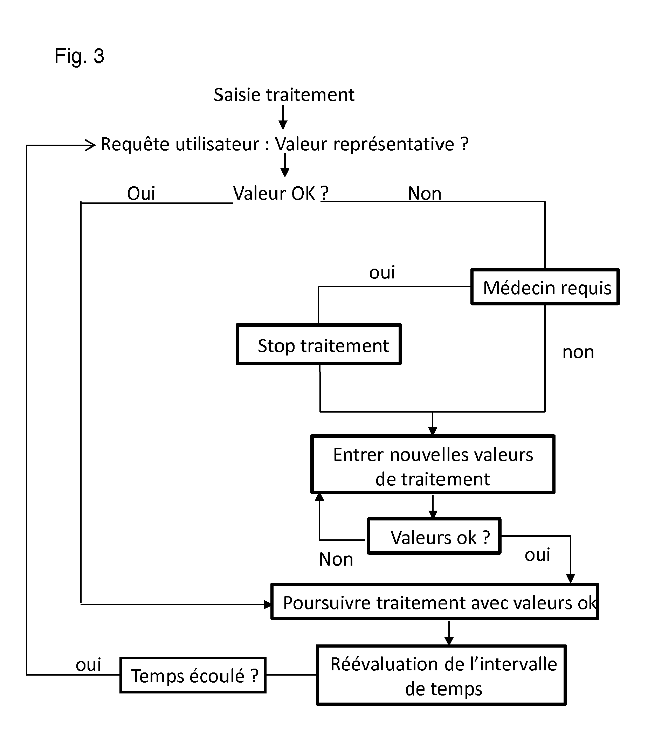

Le dispositif selon l'invention fonctionne globalement comme montré à titre d'exemple sur la figure 3, en sachant que de nombreuses variantes d'algorithme répondant au même besoin sont possibles. Le dispositif demande à l'opérateur via l'interface utilisateur 21 de saisir les données de traitement et la ou les valeur(s) représentative(s) du traitement à l'instant donné. L'unité de calcul utilise alors les règles et valeurs applicables placées dans la mémoire 23 pour vérifier si les données saisies sont acceptables. Si c'est le cas, l'unité de calcul 22 envoie l'information aux moyens de commande des pompes 24 qui alimentent alors celles-ci pour produire les débits demandés ou calculés. Si ce n'est pas le cas, l'unité de calcul 22 vérifie d'abord si l'écart est à ce point important qu'un médecin doive être appelé, auquel cas elle peut en informer l'interface utilisateur 21 qui affichera un message indiquant la nécessité d'un contrôle par le médecin et arrêtera le traitement s'il est en cours, c'est-à-dire qu'il indiquera aux moyens de commande 24 d'envoyer une consigne nulle aux pompes 70, 80, 90, 100 et selon les cas 40 et/ou 60. Sinon, le dispositif demande que les valeurs de traitements soient ajustées en respectant les règles d'acceptation applicables. Lorsque les valeurs de traitement ont été ajustées et sont acceptables le traitement peut continuer, le prochain intervalle de temps pour le contrôle suivant étant réévalué. Une fois celui-ci écoulé, le dispositif demande à nouveau de saisir la ou les valeur(s) représentative(s) et recommence son cycle d'acceptation.

La réévaluation de l'intervalle de temps est nécessaire pour d'une part assurer la sécurité du patient et d'autre part minimiser le nombre de mesures des valeurs représentatives qui nécessitent du temps, un financement et le plus souvent des prélèvements sanguins. Elle est définie par des règles et des valeurs de référence enregistrées dans la mémoire 23. Par exemple, au début du traitement, l'intervalle est égale à une demi-heure la première heure, puis une heure pour l'heure suivante puis toutes les quatre heures jusqu'à vingt-quatre heures de traitement puis toutes les douze heures. Ce programme fixe peut être modulé, par exemple après un changement significatif défini comme étant une variation d'au moins 30% de l'un des paramètres de traitement on peut imaginer revenir à un intervalle de temps d'une heure avant de passer à nouveau à quatre puis à douze heures. De la même manière, on peut aussi modifier cet intervalle de temps en fonction des valeurs reçues avec, par exemple, si le calcium mesuré est au-delà d'une limite connue qui serait utilisée pour prévenir le médecin un retour à deux mesures effectuées toutes les demi-heures avant de reprendre avec des intervalles à une, quatre et douze heures. Les valeurs fournies ci-dessus le sont à titre d'illustration du propos et le dispositif selon l'invention permet en variante qu'elles soient modifiables par exemple comme paramètre technique ajusté à la livraison du dispositif en fonction des requis du centre qui utilisera le dispositif, ou comme paramètres liés à la définition du traitement ou comme paramètres modifiés par l'opérateur en cours de traitement pour allonger ou raccourcir l'intervalle prédéterminé, ceci en fonction de ses propres connaissances.

Les valeurs saisies à ces intervalles déterminés par le dispositif sont utilisées par les règles d'acceptation des valeurs de traitement. Comme valeur(s) saisie(s), on peut à titre d'exemple considérer le calcium ionisé du patient, son rapport avec le calcium total, le calcium ionisé en entrée et/ou sortie des moyens de purification 5 ou encore le pH du patient. En intégrant ces valeurs dans les règles de calcul, l'unité de calcul 22 peut alors déterminer d'une part, si les paramètres de traitement sont dans des plages acceptables et d'autre part, l'intervalle de temps pour le prochain contrôle ainsi que les messages à afficher. Le dispositif permet ainsi de prévenir la saisie de valeurs de traitement qui peuvent mener à des déséquilibres et donc des risques pour le patient ou de coagulation du sang dans la boucle de circulation 1, 2, 3.

Un avantage essentiel de cette invention réside dans le fait qu'elle permet, par rapport aux dispositifs existants, de réduire significativement les risques liés à l'usage du citrate comme anticoagulant, l'opérateur ayant l'obligation à des intervalles de temps déterminés d'insérer une ou plusieurs valeurs déterminantes pour les règles d'acceptation. De plus elle permet de faire varier de manière sûre les paramètres de traitement tout en aidant le personnel soignant et en limitant les risques et la portée d'erreurs potentielles.

Les variantes de réalisation selon l'invention comprennent bien sûr les différentes configurations de circuit d'épuration extracorporelle connus mais aussi la possibilité d'utiliser différents solutés comprenant du citrate et du calcium et de les injecter à différents endroits du circuit. Par exemple, la solution de prédilution contenue dans le réservoir 10' peut contenir le citrate utilisé comme anticoagulant ou le dialysat contenu dans le réservoir 9' peut contenir le calcium utilisé pour restaurer la calcémie du patient. Comme autre exemple, le soluté contenant du citrate peut utiliser une formule quelconque de citrate de sodium ou du citrate-dextrose (ACD) alors que le calcium est injecté sous forme concentrée de chlorure de calcium ou de gluconate de calcium ou avec une concentration physiologique contenue dans du dialysat ou du liquide de substitution. Les concentrations de citrate et de calcium peuvent évidemment varier d'un fluide à l'autre et devront donc être saisies via l'interface utilisateur 21 afin que les masses injectées et rejetées de la boucle de circulation 1, 2, 3 puissent être évaluées par l'unité de calcul 22. D'autres électrolytes peuvent également être considérés dans le modèle, notamment le sodium injecté avec le citrate en cas d'utilisation de citrate de sodium ou le chlore mélangé à la solution de calcium. En effet, une hypernatrémie, résultant d'une injection importante de sodium, peut entrainer de graves lésions au cerveau par déchirure des vaisseaux méningés alors que le chlore est lui un élément majeur intervenant dans la détermination du pH. De même, la présence de magnésium dans des solutés injectés dans la boucle de circulation 1, 2, 3 peut être prise en compte puisque le magnésium se lie au citrate et réduit d'autant la capacité de ce dernier à se lier au calcium.

## Revendications

1. Dispositif d'épuration du sang d'un patient comprenant une boucle de circulation extracorporelle du sang (1, 2, 3), des moyens de contrôle de débit (11) pour gérer la circulation du sang dans la boucle de circulation extracorporelle du sang (1, 2, 3), des moyens d'épuration du sang (5) agencés sur la boucle de circulation extracorporelle du sang (1, 2, 3), des moyens d'injection de citrate (4) agencés pour injecter une solution comprenant du citrate dans la boucle de circulation extracorporelle du sang (1, 2, 3) et une unité de commande (20) agencée pour commander lesdits moyens d'injection de citrate (4) et lesdits moyens de contrôle de débit (11), **caractérisé par le fait que** l'unité de commande (20) est en outre agencée pour requérir à des intervalles de temps déterminés la saisie d'au moins une valeur représentative de l'état du dispositif et/ou du patient, vérifier l'adéquation de ladite valeur représentative avec des règles préétablies enregistrées dans l'unité de commande (20) et commander lesdits moyens d'injection de citrate (4) et/ou les moyens de contrôle de débit (11) selon le résultat de ladite vérification et lesdites règles préétablies.

2. Dispositif selon la revendication 1, **caractérisé en ce que** ladite valeur représentative est choisie parmi les valeurs suivantes prises seule ou en combinaison : le taux de calcium ionisé du patient soumis à un traitement par le dispositif, le ratio calcium ionisé/calcium total dudit patient, le taux de calcium ionisé pris en un point de la boucle de circulation extracorporelle du sang (1, 2, 3), le pH dudit patient.

3. Dispositif selon l'une des revendications précédentes, **caractérisé en ce que** le dispositif comprend en outre des moyens de prédilution (10) agencé en amont des moyens d'épuration du sang (5) et permettant d'injecter dans la boucle de circulation du sang (1, 2, 3) un liquide de prédilution, l'unité de commande (20) étant également agencée pour commander lesdits moyens de prédilution (10) selon le résultat de ladite vérification et lesdites règles préétablies.

4. Dispositif selon l'une des revendications précédentes, **caractérisé par le fait que** le dispositif comprend en outre des moyens de postdilution (7) agencé en aval des moyens d'épuration (5) et permettant d'injecter dans la boucle de circulation du sang (1, 2, 3) un liquide de postdilution, l'unité de commande (20) étant également agencée pour commander lesdits moyens de postdilution (7) selon le résultat de ladite vérification et lesdites règles préétablies.

5. Dispositif selon l'une des revendications précédentes, **caractérisé par le fait que** le dispositif comprend en outre des moyens d'extraction (8) permettant d'extraire la solution polluée en aval des moyens d'épuration (5), l'unité de commande (20) étant également agencée pour commander lesdits moyens d'extraction (8) selon le résultat de ladite vérification et lesdites règles préétablies.

6. Dispositif selon l'une des revendications précédentes, **caractérisé par le fait que** le dispositif comprend en outre des moyens d'injection de dialysat (9) permettant d'injecter un dialysat dans la boucle de circulation extracorporelle du sang (1, 2, 3), l'unité de commande (20) étant également agencée pour commander lesdits moyens d'injection de dialysat (9) selon le résultat de ladite vérification et lesdites règles préétablies.

7. Dispositif selon l'une des revendications précédentes, **caractérisé par le fait qu'**il comprend en outre des moyens d'injection de calcium et/ou de magnésium (6) permettant d'injecter une solution comprenant du calcium et/ou du magnésium dans la boucle de circulation extracorporelle du sang (1, 2, 3), en aval des moyens d'épuration (5) s'ils sont présents, et **par le fait que** l'unité de commande (20) est également agencée pour commander lesdits moyens d'injection de calcium et/ou de magnésium (10) selon le résultat de ladite vérification et lesdites règles préétablies.

8. Dispositif selon l'une des revendications précédentes, **caractérisé par le fait qu'**au moins une des règles préétablies enregistrées dans l'unité de commande (20) empêche les moyens d'injection de citrate (4) d'injecter dans la boucle de circulation du sang (1, 2, 3) une quantité de citrate supérieure à une première valeur limite, correspondant au seuil de tolérance du patient.

9. Dispositif selon l'une des revendications précédentes, **caractérisé par le fait qu'**au moins une des règles préétablies enregistrées dans l'unité de commande (20) empêche les moyens d'injection de citrate (4) d'injecter dans la boucle de circulation du sang (1, 2, 3) une quantité de citrate inférieure à une seconde valeur limite, correspondant au seuil en dessous duquel il existe un risque de coagulation du sang dans la boucle de circulation extracorporelle du sang (1, 2, 3).

10. Dispositif selon l'une des revendications précédentes, **caractérisé par le fait que** l'unité de commande (20) est en outre agencée pour réévaluer à chaque vérification la durée des intervalles de temps déterminés entre chaque requête de saisie d'une valeur représentative.
